(19) Europäisches Patentamt
European Patent Office
Office européen des brevets

(11) Veröffentlichungsnummer: **0 217 270**
**B1**

(12) # EUROPÄISCHE PATENTSCHRIFT

(45) Veröffentlichungstag der Patentschrift: **07.11.90**

(51) Int. Cl.⁵: **A61K 6/10**

(21) Anmeldenummer: **86113072.2**

(22) Anmeldetag: **23.09.86**

(54) **Staubfreie Alginat-Abformmassen.**

(30) Priorität: **02.10.85 DE 3535132**

(43) Veröffentlichungstag der Anmeldung:
**08.04.87 Patentblatt 87/15**

(45) Bekanntmachung des Hinweises auf die Patenterteilung:
**07.11.90 Patentblatt 90/45**

(84) Benannte Vertragsstaaten:
**AT BE CH DE FR GB IT LI NL SE**

(56) Entgegenhaltungen:
**EP-A- 0 170 020**
**FR-A- 2 553 999**
**GB-A- 1 344 377**

(73) Patentinhaber: **BAYER AG,**
**D-5090 Leverkusen 1 Bayerwerk(DE)**

(72) Erfinder: **Schwabe, Peter, Dr., Dudweiler Strasse 17,**
**D-5090 Leverkusen 1(DE)**
Erfinder: **Voigt, Reiner, Dipl.-Ing.,**
**Gustav-Radbruch-Strasse 14, D-5090 Leverkusen 3(DE)**

**EP 0 217 270 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Abformmaterialien auf Alginatbasis, ihre Herstellung und ihre Verwendung zur Herstellung von Abdrücken, bevorzugt im Dentalbereich.

Bei der Herstellung von Inlays, Kronen, Brücken und Prothesen im Zahnbereich stellt man mit Hilfe von Abformmassen ein Negativ des interessierenden Bereichs her, das anschließend mit Modellgips ausgegossen wird. Anhand des Gipsmodells kann man dann die gewünschten Anpassungen vornehmen.

Abformmaterialien aus Alginaten werden seit langer Zeit zur Herstellung von Abdrücken im Dentalbereich verwendet (US 2 345 255). Um ein Stauben des Pulvers zu verhindern, wird das Alginatpulver mit speziellen Polymeren, wie Polypropylenglykol, beschichtet (US 4 394 192). Aus der FR-A 2 553 999 sind Zahnabdruckmassen bekannt geworden, die aus einem Alginat, einem Geliermittel, einem Regler, ggf. einem Füllstoff und anderen Additiven, zumindest einer hydrophoben Flüssigkeit mit einem Dampfdruck von nicht mehr als 3,15 mm Hg bei 20°C ausgewählt aus der Gruppe der Kohlenwasserstoffe (z.B. Squalan) und einem Silikonöl, das keine hydrophilen Gruppen enthält, einem Polyvinylpyrrolidon und mindestens einer Verbindung ausgewählt aus der Gruppe der Oxide, Hydroxide und Fluoride von Metallen bestehen. Bei diesen Produkten ist zwar die Staubneigung reduziert, jedoch ist die Benetzbarkeit bzw. Mischbarkeit mit Wasser nicht zufriedenstellend.

Aus der JP-59/225 104 sind Abformmassen bekannt, die aus Alginaten, Geliermittel, Regulatoren, Füllstoffen, oberflächenaktive Mittel und Kohlenwasserstoffen bestehen. Als Kohlenwasserstoffe werden Squalan, Squalin, Nonan, Decan, Undecan, Dodecan und Tridecan genannt.

Die bekannten Abformmaterialien auf Alginatbasis stauben jedoch immer noch und sind nicht voll befriedigend.

Es wurde ein Abformmaterial auf Alginatbasis gefunden, das pulverförmiges Alginat, das mit Iso-Paraffin beschichtet ist, enthält.

Die erfindungsgemäßen Abformmaterialien stauben nicht.

Es werden Iso-Paraffine der Formel

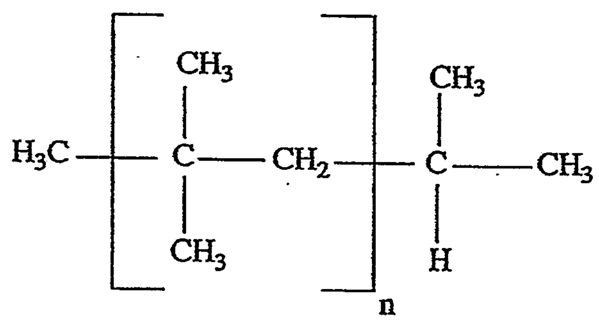

in der

n für eine der Zahlen 2, 3, 4 oder 5 steht,

für die erfindungsgemäßen Abformmaterialien bevorzugt.

Insbesondere werden Iso-Paraffine bevorzugt, bei denen n für 3 oder 4 steht.

Es ist selbstverständlich möglich, Gemische der Iso-Paraffine einzusetzen. Insbesondere bevorzugt werden 2,2,4,4,6,6,8-Heptamethyl-nonan und 2,2,4,4,6,6,8,8,10-Nonamethyl-undecan.

Die Iso-Paraffine erhält man in an sich bekannter Weise durch katalytische Oligomerisierung von Isobuten mit anschließender Hydrierung.

Die Iso-Paraffine zeigen eine Viskosität im Bereich von 4 bis 8 mPa • s/20°C und einen Siedepunkt im Bereich von 210 bis 320°C. Der Flammpunkt liegt im Bereich von 90 bis 130°C und die Zündtemperatur im Bereich von 375 bis 420°C.

Vorzugsweise enthalten die erfindungsgemäßen Abformmaterialien 0,5 bis 7 Gew.-%, insbesondere 2,5 bis 5 Gew.-%, an Iso-Paraffin.

Im allgemeinen enthalten die erfindungsgemäßen Abformmaterialien ein lösliches Alginat (z.B. das Natrium- und/oder Kaliumsalz der Alginsäure, in einer Menge von vorzugsweise 8 bis 25 Gew.-%, insbesondere 10 bis 17 Gew.-%, eine Metallverbindung, die mit Alginsäure ein wasserunlösliches Salz bildet (z.B. Blei-, Calcium- oder Magnesiumverbindungen wie Magnesiumoxid, Magnesiumcarbonat oder Calciumsulfat) in einer Menge von vorzugsweise 5 bis 40 Gew.-%, insbesondere von 10 bis 25 Gew.-%, ein Verzögerer für die Aushärtung (z.B. Alkaliphosphat, -di-phosphat, oder -polyphosphat) in einer Menge von vorzugsweise 0,5 bis 10 Gew.-%, insbesondere 1 bis 5 Gew.-%, sowie Füllstoffe (z.B. Gips, Kieselgur, Diatomeenerde, Ton, Talk usw.) in einer Menge von vorzugsweise 25 bis 85 Gew.-%, insbesondere 40 bis 75 Gew.-%. Gegebenenfalls können weitere Zuschlagstoffe wie z.B. Farb- und Geschmackstoffe, sowie die Verträglichkeit mit Gips verbessernde Verbindungen (z.B. Kaliumfluortitanat oder Kaliumfluorzirkonat) in der Abformmasse enthalten sein.

Die erfindungsgemäßen Abformmaterialien können hergestellt werden, indem man dem pulverförmigen Alginat oder gegebenenfalls einem pulverförmigem Gemisch der obengenannten Komponenten das Iso-Paraffin, vorzugsweise bei Raumtemperatur (10 bis 30°C), zusetzt. Es ist jedoch auch möglich, nur die eine oder einige der Komponenten mit Iso-Paraffin zu behandeln und dann den restlichen Komponenten zuzugeben. Der Zusatz des Iso-Paraffins erfolgt zweckmäßigerweise in einem Schaufelmischer, z.B. ein Lödige-Mischer, dessen Wand mit Düsen versehen ist, durch welche das Iso-Paraffin auf das pulverförmige Gemisch aufgesprüht wird.

Die erfindungsgemäßen Abformmaterialien zeichnen sich durch eine größere Zeichnungsschärfe und schnellere Dispergierbarkeit in Wasser aus. Sie sind geschmeidig und lassen sich nach dem Aushärten leicht vom Zahn lösen.

Beispiele

Eine Alginat-Abformmasse wurde durch Vermischen folgender Komponenten hergestellt:
Kaliumalginat 15,0%
$CaSO_4 \cdot 2H_2O$ 10,6%
$MgCO_3$ 1,5%
$Na_4P_2O_7$ 1,5%
$K_2TiF_6$ 3,4%
Kaolin 10,0%
Talk 2,1%
Diatomeenerde 55,9%

Das pulverförmige Material wurde in mehrere Portionen geteilt, die in folgender Weise mit verschiedenen Beschichtungsmitteln behandelt wurden: Das Gemisch wurde in einem Pulvermischer (Pflugscharmischer mit 3 l Volumen) vorgelegt, und von oben mittels einer Sprühpistole mit dem jeweiligen Beschichtungsmittel besprüht. Nach 10 Minuten wurde der Rührer abgestellt.

Die so beschichteten Proben wuden auf ihre relative Staubentwicklung geprüft. Das hierzu benutzte Gerät ist eine Kammer mit den Abmessungen 50 x 50 x 50 cm, welche eine Einlaßdüse und eine Absaugvorrichtung mit einem Membranfilter aufweist. Die Pumpe an der Absaugvorrichtung wird eingeschaltet und auf einen Durchfluß von 27 l/min eingestellt. Über einen an der Einlaßdüse angebrachten Probentrichter werden 400 mg Probe durch Druckluftstoß in die Kummer eingeblasen und die Pumpe 4 Minuten später abgestellt. Das Gewicht des auf dem Membranfilter abgelagerten Staubes, multipliziert mit dem empirischen Faktor 9,4, ergibt die mittlere Staubkonzentration in der Kammer.

Die Ergebnisse (Staubkonzentration in mg/m³) sind in der nachstehenden Tabelle zusammengestellt (Mittelwerte aus 5 Messungen):

| Gew.-% Beschichtungsmittel | A | B | C | D | E |
|---|---|---|---|---|---|
| 1 | | | | 263 | 376 |
| 2 | 225 | 291 | | 235 | 328 |
| 3 | 206 | 272 | 228 | 216 | 244 |
| 4 | 169 | 235 | 222 | 197 | |
| 5 | 152 | 197 | 179 | 178 | |

A 2,2,4,4,6,6,8,8,10-Nonamethyl-undecan
B 2,2,4,4,6,6,8-Heptamethyl-nonan
C Polydimethylsiloxan (100 cP/20°C) (zum Vergleich)
D Paraffinöl mit 180 cP/23°C (zum Vergleich)
E Polyethylenglykol (zum Vergleich)

Mit der Substanz C beschichtete Proben ließen sich nicht in Wasser einrühren.

**Patentansprüche**

1. Abformmaterial auf Alginat-Basis enthaltend pulverförmiges Alginat, das mit Iso-Paraffin der allgemeinen Formel

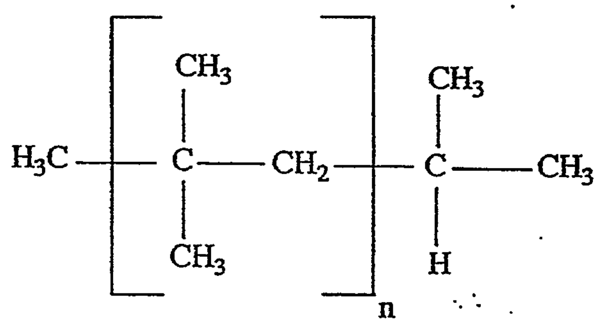

in der n für eine der Zahlen 2, 3, 4 oder 5 steht, beschichtet ist.

2. Abformmaterial auf Alginat-Basis gemäß Anspruch 1, dadurch gekennzeichnet, daß man als Isoparaffin 2,2,4,4,6,6,8-Heptamethylnonan und/oder 2,2,4,4,6,6,8,8,10-Nonanmethylundecan verwendet.

3. Abformmaterial auf Alginat-Basis gemäß Ansprüchen 1 und 2, dadurch gekennzeichnet, daß man ein Isoparaffin verwendet, das durch katalytische Oligomerisierung von Isobuten mit anschließender Hydrierung hergestellt wird.

4. Abformmaterial auf Alginat-Basis gemäß Ansprüche 1–3, dadurch gekennzeichnet, daß es 0,5–7 Gew.-% des Isoparaffines enthält.

5. Abformmaterial auf Alginat-Basis gemäß Ansprüchen 1–4, dadurch gekennzeichnet, daß es 2,5–5 Gew.-% des Isoparaffines enthält.

6. Verwendung von Abformmaterial gemäß Ansprüchen 1–5 zur Herstellung von Abdrücken in der Dentaltechnik.

**Claims**

1. Alginate-based impression material containing pulverulent alginate which is coated with an iso-paraffin of the general formula

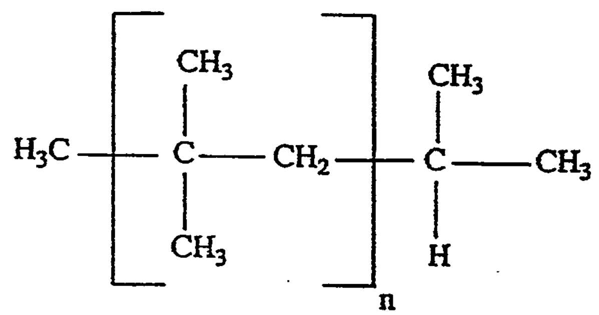

in which n represents one of the numbers 2, 3, 4 or 5.

2. Alginate-based impression material according to Claim 1, characterized in that 2,2,4,4,6,6,8-heptamethylnonane and/or 2,2,4,4,6,6,8,8,10-nonamethylundecane is used as the iso-paraffin.

3. Alginate-based impression material according to Claims 1 and 2, characterized in that an iso-paraffin which is prepared by catalytic oligomerization of isobutene with subsequent hydrogenation is used.

4. Alginate-based impression material according to Claims 1–3, characterizes in that it contains 0.5–7% by weight of the iso-paraffin.

5. Alginate-based impression material according to Claims 1–4, characterized in that it contains 2.5–5% by weight of the iso-paraffin.

6. Use of impression material according to Claims 1–5 for producing impressions in the dental field.

## Revendications

1. Matière à mouler à base d'alginate contenant un alginate en poudre qui est enduit avec une isoparaffine de formule générale:

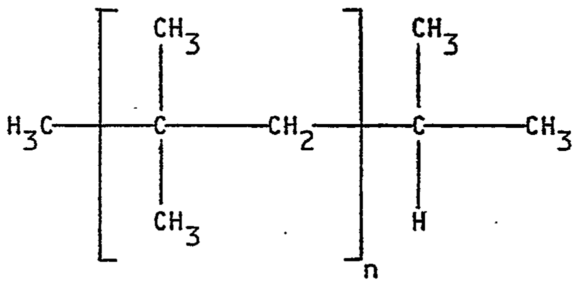

dans laquelle n est l'un des nombres 2, 3, 4 et 5.

2. Matière à mouler à base d'alginate selon la revendication 1, caractérisée en ce que l'on utilise comme isoparaffine le 2,2,4,4,6,6,8-heptaméthylnonane et/ou le 2,2,4,4,6,6,8,8,10-nonaméthylundécane.

3. Matière à mouler à base d'alginate selon les revendications 1 et 2, caractérisée en ce que l'on utilise une isoparaffine qui est fabriquée par oligomérisation catalytique d'isobutène suivi d'hydrogénation.

4. Matière à mouler à base d'alginate selon les revendications 1 à 3, caractérisée en ce qu'elle contient 0,5–7% en poids de l'isoparaffine.

5. Matière à mouler à base d'alginate selon les revendications 1 à 4, caractérisée en ce qu'elle contient 2,5 à 5% en poids de l'isoparaffine.

6. Utilisation de la matière à mouler selon les revendications 1 à 5 pour la fabrication d'empreintes dans la technique dentaire.